Europäisches Patentamt

(19) European Patent Office

Office européen des brevets

(11) Publication number : **0 506 793 B1**

## (12) EUROPEAN PATENT SPECIFICATION

(45) Date of publication of patent specification :
**02.03.94 Bulletin 94/09**

(21) Application number : **91901714.5**

(22) Date of filing : **12.12.90**

(86) International application number :
**PCT/DK90/00327**

(87) International publication number :
**WO 91/09989 11.07.91 Gazette 91/15**

(51) Int. Cl.⁵ : **A23L 1/236**, A23G 3/30, A23G 3/00, A61K 9/68, A61K 9/36

(54) **A METHOD FOR APPLYING A HARD COATING ON CORES OF ADIBLE OR CHEWABLE MATERIAL AND COATED MATERIAL OBTAINED BY THE METHOD.**

(30) Priority : **21.12.89 DK 6544/89**

(43) Date of publication of application :
**07.10.92 Bulletin 92/41**

(45) Publication of the grant of the patent :
**02.03.94 Bulletin 94/09**

(84) Designated Contracting States :
**AT BE CH DE DK ES FR GB GR IT LI LU NL SE**

(56) References cited :
**WO-A-88/08671**
**GB-A- 2 115 672**
**US-A- 4 238 510**
**US-A- 4 317 838**
**US-A- 4 423 086**
**US-A- 4 753 790**

(73) Proprietor : **DTF HOLDING A/S**
**Dandyvej Postbox 208**
**DK-7100 Vejle (DK)**

(72) Inventor : **DYHR, Hans, Henrik**
**Billensteinsvej 33**
**DK-7120 Vejle Ost (DK)**
Inventor : **SORENSEN, Lene, Bothilde,**
**Ostergaard**
**Barrit Langgade 96**
**DK-7150 Barrit (DK)**

(74) Representative : **Zeuthen-Aagaard, Henrik et al**
**Chas. Hude, 33 H.C. Andersens Boulevard**
**DK-1553 Copenhagen V (DK)**

Jouve, 18, rue Saint-Denis, 75001 PARIS

## Description

### Technical Field

The present invention relates to a method for applying a hard sorbitol coating on cores of edible or chewable material, where the cores in a moving bed in a coating apparatus are subjected to a number of coating cycles, each of the cycles comprising applying, distributing and smoothing out sorbitol-containing material and drying by means of drying air, whereupon the coated cores are optionally polished, as well as coated material obtained by the method.

### Background Art

It has been known for a long time to coat edible and chewable materials with a sweet coating. Earlier such sweet coatings were usually based on sugar. In recent years a wish to make coated materials with sweet, sugarless coatings, for instance based on sorbitol as a sweetener has developed.

Within traditional sugar based coating techniques a distinction is made between different coating methods, for instance soft coating and hard coating. In principle, soft coating is based on the formation of a coating without substantial evaporation of water from the coating material added. This is done by adding both syrup or sugar solution and a dusting powder, whereby the material added forms a relatively soft coating on the cores without evaporation of water. In hard coating only a syrup or sugar solution is added, after the evaporation of water forming a coating harder than the one formed by soft coating. As a consequence of the water evaporation required, hard coating is usually more time-consuming.

The crystallization of sorbitol is encumbered with problems due to the fact that there are more unstable crystal forms. In crystallization of sorbitol from a solution an unstable amorphous form is firstly formed, said amorphous form subsequently being transformed via an unstable $\alpha$-crystal form and an unstable $\beta$-crystal form to a stable gamma-crystal form. The final gamma-crystal structure is the one desired in a hard coating, as it ensures that the coating is provided with a pleasant crunchy consistency.

When moving from the traditional sugar-based coating technique to sugarless, for instance sorbitol-based, coating, difficulties arise because sorbitol is difficult to crystallize, especially if the hard and crunchy gamma-crystal structure is desired.

Several methods are already known for hard coating of cores of edible or chewable material with a sorbitol coating. Thus, WO Publication No. 81/01100 discloses a method for hard coating with sorbitol, where cores placed on a moving bed are provided with a syrup having a concentration of dry matter of between 60 and 85% by weight and with a D-sorbitol content based on dry matter of more than 80%, preferably more than 95%, and a temperature below 55°C. In this method, the sorbitol syrup must have a degree of saturation of between 0.65 and 1.25, preferably between 0.5 and 1.15 when the sorbitol syrup is contacted with the cores to be coated. The coating is carried out in a conventional manner by means of succesive cycles, said cycles comprising a first step with the addition of the sorbitol syrup to the bed of cores and a second step wherein the addition is stopped while the rotation and the prevailing temperature are maintained. The said second step is continued until the material applied during the first step is distributed over the cores and dried. The number of cycles determines the thickness of the coating obtained, there being narrow limits to the thickness of the layer applicable in each cycle if the coated product obtained is to have the desired attractive appearance. Compared to the use of sugar for coating it is substantially more difficult to make a satisfactory sorbitol-coated product, because it is difficult to crystallize sorbitol to form the desired crystalline coating. To this should be added that chewing gum cores which are subjected to a coating step with sorbitol easily stick to each other and form undesirable clumps. These difficulties are overcome by the method described in the above WO Publication, which provides a good hard, crunchy coating, which is desirable in an organoleptic quality product. The method is, however, very time-consuming as the coating per se takes six hours to which should be added that the coated cores must be conditioned for a certain period of time before the final polishing.

US Patent No. 4 238 510 and BE Patent No. 884 317 (Cherukuri et al.) ($\equiv$ US-A-4 317 838) discloses a method for sorbitol coating wherein it is ensured that the sorbitol crystallizes. By this method, cycles are repeatedly carried out comprising (a) application of a first coating syrup containing sorbitol, an adhesion or binder component and a film-forming agent, (b) application of a dry dusting powder in the form of a mix comprising sorbitol in a powdered form, a moisture absorbing component, an anti-sticking component and a dispersing agent, (c) distributing and smoothing out of the material applied and (d) drying of the layer of material formed on the cores. Following said treatment it is necessary to condition the coated cores for 1 to 3 days to ensure the crystallization. After the conditioning another coating syrup is applied, said syrup containing the same components as those contained in the powder mix but dispersed or dissolved in water. Said conditioning for 1 to 3 days takes place on trays and the transfer of the coated cores from the coating apparatus to trays and back to the coating tray prior to the application of the second coating syrup is a time and labour-consuming process which results in a poor utilization of the coating apparatus. Furthermore, the drying time required in step (d) of the drying cycles is comparatively long which constitutes a further limitation to the capacity of the coating apparatus. In spite of the extensive time-consumption by the known method, the coating layer is not as crunchingly hard as the coating layer obtained according to WO 81/01100. This is however not aimed at, as a soft coating is desired.

US Patent No. 4 317 838 and GB Patent No. 2 115 672 (Cherukuri et al.) disclose a further development of the method disclosed in US Patent No. 4 238 510, wherein more detailed instructions as to the composition of the material applied and the use of drying air are given. Said method is more simple but large amounts of auxiliary agents are still used. Like the product obtained according to US Patent No. 4 238 510, the resulting product has a soft crystal structure in the coating.

More examples of soft and hard coatings with sorbitol are known, for instance such as described in WO Publication No. 88/08671, JP 61249350 and JP 57107159 but these patent specifications do not state how a crispness as good or better than the crispness obtained by following the instruction of WO Publication No 81/01100 is obtained.

However, it has now been found that an even better sorbitol coating can be obtained, said coating being even more crunchingly crisp than the coating obtained according to WO 81/01100 and at the same time the coating is carried out substantially faster, whereby the coating costs are reduced drastically.

Disclosure of Invention

It is therefore the object of the present invention to provide a method for sorbitol coating by which a hard coating shell with an improved crunchy crispness is obtained and wherein the entire coating process including the subsequent polishing, is carried out in one step in the coating apparatus without intermediate conditioning and wherein a high coating rate is obtained, calculated as weight gain per surface area per time unit is obtained.

The above object is obtained by the inventive method for applying a hard sorbitol coating on cores of edible or chewable material where the cores in a moving bed in a coating apparatus are subjected to a number of coating cycles, each of the cycles comprising applying, distributing and smoothing out sorbitol-containing material and drying by means of drying air, whereupon the coated cores optionally are polished, which metod according to the invention is characterised in that in succession the coating cycles comprise:

1) a sealing wherein the cores are subjected to 1 to 6 cycles, each comprising the following steps (a), (c) and (d):

step (a), applying a sorbitol-containing suspension having a temperature of 15-25°C and containing at least 83% by weight of D-sorbitol based on dry matter,

step (c) distributing and smoothing out the applied material on the surface of the cores,

step (d), drying the material deposited on the cores using drying air having a temperature of 20-40°C and a relative humidity of 8-30%,

2) a forced hard coating, wherein the cores are subjected to one or more cycles, each comprising the steps (a), (b), (c) and (d), each step (a), (c) and (d) being as mentioned above and step (b) comprising the application of a dry sorbitol powder with a particle size of between 40 micrometer and 125 micrometer and a D-sorbitol content of at least 94% by weight based on dry matter, and

3) a hard coating, wherein the cores are subjected to one or more cycles, each comprising the above steps (a), (c) and (d).

By the method according to the invention a hard crunchy product is obtained, which in an Instron test has proved to be even more crunchingly crisp than a corresponding product prepared according to the method described in WO Publication No. 81/01100. The results of a comparative Instron test also show that by the method according to the invention an improved adhesion to the chewing gum core is obtained. The entire method, including the subsequent polishing, may be carried out in a single sequence of process steps in the same coating apparatus during a short time, for instance 100 to 160 minutes, preferably 115 to 145 minutes without intermediate conditioning. Coating may be carried out with a low content of auxiliary agents which is essential to the high organoleptic quality of the end product. A suspension containing about 10 % by weight solid auxiliary agents, for instance talc powder and titan dioxide, or less, for instance about 5 to 2% by weight may be used, it has even been found that the method may be carried out without any content of for instance talc powder as anti-sticking component or dispersing agent thereby providing particularly excellent organoleptic properties.

An essential feature of the method according to the invention is that said method is started with a sealing carried out in the same way as a conventional hard coating. The sealing is carried out with 1 to 6 cycles, preferably 3 cycles.

By the method according to the invention, step (d) may advantageously be carried out with drying air having a temperature of 26 to 30°C and a relative humidity of 9 to 15% in a quantity of 25 to 95 m³/hour per m³ of the surface area of the cores thereby ensuring the short preparation time and the desired hard, crunchy coating.

The temperature of the suspension used in step (a) is preferably 18 to 22 °C.

A slowly rotating coating apparatus with mixing blades is a suitable apparatus for carrying out the method according to the invention, said coating apparatus being provided with perforations in the cylinder-shaped surface of the coating apparatus to provide drying air. Said form ensures the required supply of drying air. Said coating apparatus may for instance be a coating apparatus of the brand Dria 1200, available from Driam Metallprodukt GmbH & CO.KG, Eriskirch, The Federal Republic of Germany. Such coating apparatus may be included in an assembly provided with a dehumidifier, for instance of the brand Munters, available from Karl Munters A/S, Farum Gydevej 89, 3520 Farum, Denmark.

The method according to the invention may advantageously be carried out in such a manner that during the sealing (1) the drying step (d) is carried out by blowing drying air in at the bottom of the drying pan and up through the bed of cores, and that during said forced hard coating (2), step (d) is carried out by blowing the drying air in from the top of the coating apparatus and down through the bed of cores, and that during said hard coating (3) step (d) is carried out by blowing the drying air in at the bottom of the coating apparatus and up through the bed of cores. As a result, a good contact is ensured between the cores and the drying air. Furthermore, the carrying along with the air of the dusting powder, which has been added during the forced hard coating, is avoided.

The invention further relates to a dragee in the form of an edible or chewable core with hard sorbitol coating prepared by the method according to the invention.

Examples of edible or chewable cores coatable by the method according to the invention include chewing gum, including medical and semimedical chewing gum, pastilles, snack products, caramel, chocolate, dried fruits, dried vegetables, fruit bars, deshelled nuts, seeds, for instance almonds, sunflower seeds or pumpkin seeds, and medical products.

An essential feature of the present invention is that the sorbitol powder used as dusting powder is pure dry D-sorbitol powder without auxiliary agents.

Thus, the content of D-sorbitol in the dusting powder is. at least 94% by weight based on dry matter, preferably at least 96% by weight, particularly preferred at least 99% by weight.

It is rather surprising that dusting powder with a low content of auxiliary agents and even entirely without auxiliary agents may be used, as a person skilled in the art on the basis of the teaching appearing from for instance Cherukuri's above specifications may get the impression that considerable quantities of auxiliary agents, such as binding agent, are needed to ensure a sufficiently good adhesion to the chewing gum core.

As the sorbitol solution to form part of the sorbitol-containing suspension applicable in step (a) by the method according to the invention, a sorbitol solution available in the trade may be used. Such sorbitol solution contains according to the specifications at least 68% by weight D-sorbitol, usually about 70% by weight. In the examples below said solution is designated as 70% by weight sorbitol solution.

The sorbitol suspension used preferably has a low content of auxiliary agents but may of course contain conventional auxiliary agents as required. Thus, the suspension may contain an anti-sticking component, for instance calcium carbonate, talc powder or magnesium trisilicate, preferably talc powder. As mentioned above, the method according to the invention may be carried out entirely without the use of an anti-sticking component or with only a low content of anti-sticking component. Substances such as talc powder or other anti-sticking components may also be used as dispersing agents but may also be left out or kept at a low level, if desired.

As mentioned, a high D-sorbitol content in the sorbitol-containing suspension applied in step (a) is essential to obtain the desired hard crunchingly crisp coating. Thus the content thereof is at least 83% by weight, preferably at least 92% by weight, particularly preferred at least 96% based on dry matter.

Contrary to this, in the case of soft coating, the sorbitol syrup used is a sorbitol solution with below 68% by weight D-sorbitol or with a higher content of additives of for instance hydrogenated disaccharides, such as maltitol, tri- and heptahydrogenated saccharides and the like. Such additives, by soft coating used in quantites of from 10 to 35% by weight, prevent or render difficult the gamma-crystal formation as they directly inhibit the crystallization. By using said additives a coating layer is obtained which contains amorphous sorbitol. Such coating is classified as a soft coating which means to say that it is somewhat less hard than the hard coating prepared by the method according to the invention.

The method according to the invention may be carried out both with and without talc powder in the sorbitol-containing suspension applied during step (a). The addition of dispersing agents such as talc powder or calcium carbonate has no adverse effect on the crispness, but if too large quantities are used, such as exceeding 11 % by weight, a substantial deterioration of the mouthfeel and thereby the entire perception takes place. By the method according to the invention the best organoleptic properties have been obtained with 2% by weight talc powder in the sorbitol-containing suspension. With quantities of talc powder of between 5 and 10% by weight the product still has a fair quality, but a somewhat poorer mouthfeel compared to the use of 2% by weight talc powder.

Colourants may be added in a conventional manner. In principle, any colourant which is approved for use in foodstuffs may be used. Examples of such colourants include titan dioxide, chlorophyll, indigotine, betacarotene or mixtures thereof. The colourants are preferably added during the final hard coating.

Furthermore, flavouring agents in the form of both liquid and solid flavouring agents of both natural and synthetic origine may be added. Examples of flavouring agents may comprise peppermint oil, spearmint oil and mixtures thereof, menthol, citrus oils, such as orange oil, lime oil or lemon oil, fruit extracts or essences, such as extract or essence of pear, apple, cherry, raspberry, strawberry, plum, cherry plum, grape, pineapple, peach, fig or date.

Corresponding synthetic flavouring agents with the same flavour may be used as a supplement or alternative to the above natural flavouring agents. The flavouring agents may be added to the coating shell at different times during the method according to the invention, for instance between 1 and 3 times during the preparation. The first time may, for instance, be just after the sealing, the second time after the third cycle of the forced hard coating, in which case it may be in the form of a powdered flavoring agent and the third time may be during the final smoothing hard coating. The quantities of flavouring agent used in the coating shell will be from 0.4 to 6% by weight, preferably 1.0% by weight calculated as a ratio of the total coating shell. Highly potent sweeteners, such as aspartame and acesulfam-K may further be added. The quantity of said sweeteners may vary between 0.03 and 0.15% by weight, preferably 0.1% by weight calculated on the basis of the total coating shell.

After the coating per se a polishing is carried out, as mentioned, said polishing by the method according to the invention may advantageously be carried out in the same coating apparatus. At the polishing a polishing agent, for instance carnauba wax/talc powder, is added in a conventional manner, for instance in a ratio of 1 to 1, carnauba wax alone, vegetable oils or solutions of gum arabic or other types of vegetable rubber.

The further scope of the applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

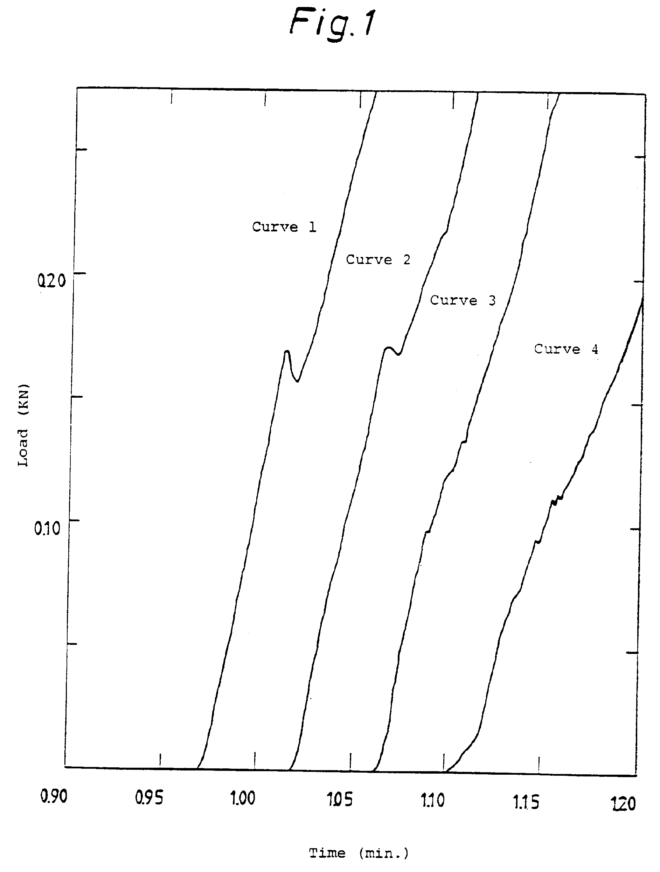

Fig. 1 illustrates the results of a comparison test between a chewing gum prepared by the method according to the invention (curves 1 and 2) and a chewing gum prepared by 5 the method disclosed in WO 81/01100 (curves 3 and 4). The test was carried out on an Instron apparatus where the load necessary to maintain a constant rate at the cutting through of the chewing gum in KN in relation to time was measured. As it appears from the curves, a substantially better, crunchy crisp product is obtained by using the method according to the invention compared to prior art.

Best Mode for Carrying Out the Invention

The invention is illustrated further below by means of some examples.

Examples

The general method in the below examples is as follows:

Chewing gum cores prepared in the form of sheets are used as starting material, said sheets being rolled out to chewing gum cores each having a weight of about 1 g by means of a profiled roller. An assembly is used comprising a coating apparatus of the brand Dria 1200, available from Driam Metallproduckt GmbH & CO.KG, Eriskirch, the Federal Republic of Germany. Dria 1200 is adapted for coating of 50 kg of chewing gum cores corresponding to a surface area of 23 $m^2 \pm 5\%$. The assembly has computer control of dosing quantites, smoothing out times, drying times, air quantities, drying air temperatures and direction of air flow. The coating apparatus may be set at different rates of speed from 1 to 15 r.p.m.

In the coating process 50 kg of chewing gum cores are filled in the coating apparatus which is set at a speed of 8 r.p.m.. During the rotation the chewing gum cores are separated from each other. Drying air is fed to the assembly and surplus talc powder added during the rolling of 5 the chewing gum cores is removed from the chewing gum cores. The separation and air flow continue for about 5 minutes.

Sealing

The rotation speed of the coating apparatus is increased to 11 r.p.m. and the first dosing of the sorbitol solution is added. The quantity of sorbitol suspension added is 0.5 kg. Smoothing out is subsequently carried out, that is 5 rotation without air flow, where the suspension applied is smoothed out on all the chewing gum surfaces for about 0.6 minute. Drying then takes place for 5 minutes by blowing air towards the coating bed, that is from below, also designated counter flow. The above process is repeated the neccesary number of times, for instance between one and six times. Said first cycles, where only sorbitol suspension is dosed, are designated sealing in the present specification.

Forced hard coating

Subsequently a new type of coating is carried out, designated forced hard coating. Said hard coating comprises the same type of cycle as the sealing but with the addition of a further step after the application of the sorbitol solution, said step involving the introduction of a dusting powder in the form of a sorbitol powder. A cycle within said part of the coating process comprises firstly the application of about 1.2 kg sorbitol suspension and immediately following the distribution of the suspension in the course of 30-60 seconds, about 2.8 kg. sorbitol powder is dusted on by means of compressed air. During the dusting of the dusting powder the rotation speed of the apparatus is reduced to 8 r.p.m.. When all the powder has been absorbed by the wet chewing gum cores after a smoothing out time of about 2 minutes, the rotation speed of the pan is increased to 11 r.p.m. and the chewing gum cores are dried by feeding drying air from the top of the chewing gum cores which is designated direct air flow or con-current flow. The drying time is about 5 minutes. This process is also repeated a suitable number of times, for instance about 5 times, until the desired weight increase has been obtained.

Hard coating

The coating process per se is completed by a conventional hard coating, said hard coating in principle being carried out in the same manner as the sealing. The rotation speed of the pan is 11 r.p.m. and the rotation speed is kept constant during the remaining portion of the process. The sorbitol suspension is added in decreasing quantity, starting with 1.2 kg. per cycle. The smoothing out time is adapted thereto to ensure an even distribution. The air flow is reversed to counter flow and the drying time is about 5 minutes per cycle. Said process is repeated till the required final weight has been obtained, usually 8 to 10 cycles.

Polishing

The coated cores are then polished with a polishing agent comprising carnauba wax/talc powder in a weight ratio of 1 to 1. 0.5 g polishing agent is used for 1 kg of coated cores. After the addition of the polishing agent, said polishing agent is distributed during rotation for 5 minutes. Drying air of a temperature of 10°C is then fed for 10 to 15 minutes. The coated chewing gum cores are now finished and ready for packing.

To provide the required low humidity of the drying air a dehumidifier of the brand Munters, available from Karl Munters A/S, Farum Gydevej 89, 3520 Farum, Denmark, is used.

The 70% by weigth sorbitol solution used in the examples contains according to an analysis 71.25% by weight D-sorbitol.

Example 1

The present example illustrates hard coating of chewing gum cores with sorbitol by the method according to the invention.

50 kg of conventional chewing gum cores each having a weight of 1 g and corresponding to a total surface area of 23 $m^2 \pm 5\%$ are coated using the following materials:

Suspension AI

| | |
|---|---|
| 70% by weight sorbitol solution | 97.6% by weight |
| Talc powder | 2.0% by weight |
| Titan dioxide | 0.4% by weight |

Suspension AII:

| 70% by weight sorbitol solution | 94.6 % by weight |
|---|---|
| Talc powder | 2.0% by weight |
| Titan dioxide | 0.4% by weight |
| Water | 3.0% by weight |

Powder

The sorbitol dusting powder has a purity of more than 99 % by weight D-sorbitol and a particle size of 40 micrometer to 125 micrometer. Said dusting powder is used without auxiliary agents.

The coating is carried out as described below during a total of 17 cycles finishing with the polishing.

Cycle 1-3, sealing

The coating comprises firstly a sealing of the cores by means of three coating cycles each comprising the application of 0.5 kg suspension AI having a temperature of 20°C ± 2°C, smoothing out for 30 seconds and drying for 5 minutes. The drying is carried out in counter flow. The drying air has a temperature of 28°C ± 2°C and a dew point of -7°C corresponding to 2.25 g water/kg air or a relative humidity of 9.8%. The drying air is fed at a quantity of 25 m³/minute corresponding to 1500 m³/hour. Total time for the first three coating steps is 16 minutes.

Cycle 4-8, forced hard coating

A coating layer is subsequently applied by in the order for each cycle
a) applying suspension AII having a temperature of 20°C ± 2°C,
b) applying powder,
c) distributing and smoothing out, and
d) drying under the same conditions as during the sealing in cycle 1-3, apart from the drying air being fed in concurrent flow.
The quantities applied and the smoothing out and drying times are as follows.

| Cycle | Suspension AII (kg) | Powder (kg) | Smoothing out time (min) | Drying time (min) |
|---|---|---|---|---|
| 4 | 0.6 | 2.5 | 1.0 | 5.0 |
| 5 | 1.0 | 1.3 | 1.0 | 5.0 |
| 6 | 1.0 | 2.0 | 1.0 | 5.0 |
| 7 | 1.2 | 2.7 | 2.0 | 5.0 |
| 8 | 1.2 | 2.7 | 2.0 | 4.0 |

Total time consumption for cycles 4 to 8 is 37 minutes.

Cycle 9-17, hard coating

The coating is completed with a hard coating, each cycle comprising
a) applying suspension AII, with a temperature of 20°C ± 2°C
c) distributing and smoothing out, and
d) drying in counter flow.
In the individual cycles the following conditions are used:

7

| Cycle | Suspension AII (kg) | Smoothing out time (min) | Drying time (min) |
|---|---|---|---|
| 9 | 1.2 | 0.6 | 5.0 |
| 10-11 | 1.0 | 1.0 | 5.0 |
| 12-13 | 1.0 | 1.5 | 5.0 |
| 14-16 | 0.7 | 1.3 | 5.0 |
| 17 | 0.5 | 5.0 | |

The total time for cycles 9-17 is 57 minutes. The total time from step 1 to and including step 17 is thus 11o minutes.

Subsequently the coated cores are polished by means of a polishing agent comprising carnauba wax/talc powder in the weight ratio of 1 to 1. 0.5 g of the polishing agent is used for 1 kg of coated cores. After the addition of the polishing agent, said polishing agent is distributed for 5 minutes during rotation of the coating apparatus. Drying air having a temperature of 10°C is fed for 10-15 minutes.

In said manner a smooth, coated chewing gum with a hard crunchy shell, polished and ready for packing is obtained. The finished product has a good harmony between sweetness and taste and at the same time a pleasant mouthfeel. Total coating time including the subsequent polishing in the coating apparatus is 130 minutes.

Example 2

Substantially the same procedure as in Example 1 is repeated using the same sorbitol dusting powder as in Example 1 and suspension AII for both the sealing, the forced hard coating and the conventional hard coating.

The coating is carried out as described below during a total of 17 cycles finishing with polishing.

Cycle 1-3, sealing

In each of the three coating cycles 0.5 kg. suspension AII is applied at a temperature of 20°C ± 2°C and smoothing out is carried out for 40 seconds and drying for 5 minutes. The drying is carried out in counter flow with a quantity of air, temperature and humidity as in Example 1. Total time for the first 3 coating cycles is 15 minutes.

Cycle 4-8, forced hard coating

Said coating cycles are carried out as in Example 1, the quantities applied and the smoothing out and drying times being as follows:

| Cycle | Suspension AII (kg) | Powder (kg) | Smoothing out time (min) | Drying time (min) |
|---|---|---|---|---|
| 4 | 1.0 | 2.6 | 1.5 | 5.0 |
| 5 | 1.2 | 2.8 | 2.0 | 5.0 |
| 6 | 1.2 | 2.8 | 2.0 | 5.0 |
| 7 | 1.2 | 2.8 | 2.0 | 5.0 |
| 8 | 1.2 | 2.8 | 2.0 | 5.0 |

Total time consumption for cycles 4-8 is 40 minutes.

Cycles 9-17, hard coating

The hard coating is carried out in the same manner as in Example 1, the following conditons being used in the individual cycles.

| Cycle | Suspension AII (kg) | Smoothing out time (min) | Drying time (min) |
|---|---|---|---|
| 9 | 1.2 | 1.0 | 5.0 |
| 10-12 | 1.0 | 1.5 | 5.0 |
| 13-14 | 0.8 | 1.5 | 5.0 |
| 15-16 | 0.7 | 1.3 | 5.0 |
| 17 | 0.5 | 1.0 | 3.0 |

Total time consumption for cycles 9-17 is 60 minutes. The total time from step 1 to and including step 17 is thus 115 minutes.

The coated cores are then polished in the same manner as described in Example 1. Total coating time including the subsequent polishing in the coating apparatus is 130 minutes.

The finished product is an even and smooth dragee with a hard crunchy shell, polished and ready for packing. The finished product has a crunching, crisp initial chew with a good sweetness, mouthfeel and aroma release.

Example 3

The coating described in Example 2 is repeated using the same sorbitol dusting powder but using suspension B instead of suspension AII.

Suspension B

| | |
|---|---|
| 70% by weight sorbitol solution | 96.6% by weight |
| Titan dioxide | 0.4% by weight |
| Water | 3.0% by weight |

The coating including the polishing may be carried out in 130 minutes.

The finished product has a good harmony between sweetness and taste and at the same time a pleasant mouthfeel. The coating is crisp and crunchy.

Example 4

The coating described in Example 2 is repeated using the same sorbitol dusting powder but using suspension C instead of suspension AII.

Suspension C

| | |
|---|---|
| 70% by weight sorbitol solution | 95.6% by weight |
| Talc powder | 1.0% by weight |
| Titan dioxide | 0.4% by weight |
| Water | 3.0% by weight |

The coating including the polishing may be carried out in 120 minutes.

The finished product has a good harmony between sweetness and taste and at the same time has a pleasant mouthfeel. The coating is crisp and crunchy.

### Example 5

The coating described in Example 2 is repeated using the same sorbitol dusting powder but using suspension D instead of suspension AII.

### Suspension D

| | |
|---|---|
| 70% by weight sorbitol solution | 91.6% by weight |
| Talc powder | 5.0% by weight |
| Titan dioxide | 0.4% by weight |
| Water | 3.0% by weight |

The coating including the polishing may be carried out in 115 minutes.

The product is smooth, completely coated and with a hard crunchy shell. There is a good harmony between sweetness and taste but in an organoleptic evaluation of the product the talc powder content is perceived as a less pleasant, floury mouthfeel.

### Example 6

The coating described in Example 2 is repeated using the same sorbitol dusting powder but using suspension E instead of suspension AII.

### Suspension E

| | |
|---|---|
| 70% by weight sorbitol solution | 85.8% by weight |
| Talc powder | 10.0% by weight |
| Titan dioxide | 0.2% by weight |
| Water | 4.0% by weight |

The coating including the polishing may be carried out in 130 minutes.

The product is smooth, completely coated and with a hard crunchy shell. There is a good harmony between sweetness and taste but in an organoleptic evaluation of the product the talc powder content is perceived as a less pleasant, floury mouthfeel to an even greater extent than the coated chewing gum prepared according to Example 5.

### Example 7

The present Example illustrates hard coating of almonds with sorbitol by the method according to the invention.

50 kg of almonds are filled into the coating apparatus and the rotation is started at a speed of 10 r.p.m.. The computer is set at the required values: dosing quantites, smoothing out times, drying times, air quantity, temperature of drying air and direction of air flow. The computer is also set at the number of cycles to be run during sealing, forced hard coating and the final hard coating, respectively.

The suspension used has the following composition:

| | |
|---|---|
| 70% by weight sorbitol solution | 95.5% by weight |
| Titan dioxide | 0.3% by weight |
| Water | 4.0% by weight |
| Indigotine | 0.2% by weight |

Sealing: 5 cycles.

Forced hard coating: 8 cycles are used which is necessary to form an even surface capable of covering the rather uneven almonds.

Hard coating: 7 cycles providing a smooth hard coating.

Total coating time: 130 minutes.

Flavouring is added to the coating shell in the form of peppermint oil added to the last sealing layer.

The almonds are not polished as a surface is desired which is a little rough and feels slightly dusty.

The end product is of a dusty light blue colour and the almonds are evenly rounded and slightly dusty.

Suspension temperature: 24°C ± 5°C.

Temperature of drying air: 26°C ± 2°C

Relative humidity: 19% (at 26°C)
Quantity of air: 36 m³/minute.

<u>Example 8</u>

The present example illustrates hard coating of dried banana slices with sorbitol by the method according to the invention.

40 kg of dried banana slices are filled into the coating apparatus. The density being low, there is only space for 40 kg of dried banana slices in the coating apparatus. At the same time there is a large surface area. The same method is used as the one used when coating almonds.

The suspension comprises:

| | |
|---|---|
| 70% by weight sorbitol solution | 98.7% by weight |
| Titan dioxide | 0.1% by weight |
| Water | 1.0% by weight |
| Caramel colourant (E150) | 0.2% by weight |

Suspension temperature: 20°C ± 2°C
Temperature of drying air: 28°C ± 2°C
Relative humidity: 10% (at 28°C)
Quantity of air: 24 m³/minute.
Chocolate flavouring is added to the shell as flavouring.

A low quantity of air is kept as the density of the product is low. Very low humidity is used to prevent the banana slices from turning soft. Polishing is carried out with carnauba wax/talc powder in a weight ratio of 1 to 1.

The product presents itself as crunchy, crisp pieces with a brown surface.

<u>Example 9</u>

The present example illustrates hard coating throat lozenges with sorbitol by the method according to the invention.

50 kg of throat lozenges (salt/liquorice) in the form of balls with a diameter of about 8 mm are filled into the 5 coating apparatus. Air having a temperature of about 15°C is fed in counter flow to immediately prevent the balls from sticking together. The same number of cycles as in Example 1 is used,

The suspension used comprises:

| | |
|---|---|
| 70% by weight sorbitol solution | 97.6% by weight |
| Titan dioxide | 0.4% by weight |
| Water | 2.0% by weight |

Suspension temperature: 20°C ±2°C
Temperature of drying air: 22°C ±2°C
Quantity of drying air: 36 m³/minute
Relative humidity: 9% (at 22°C)
Liquid liquorice flavour is added to the shell as flavouring.

The throat lozengers are polished after completed coating with carnauba wax/talc powder in the weight ratio of 1 to 1.

<u>Comparison tests</u>

A test was carried out at Bioteknisk Institut in Kolding, Denmark to test the sorbitol shell of a product according to the invention compared to a product prepared according to WO Publication No. 81/01100, that is ordinary coating without powder. The results appear from Fig. 1 where the curves 1 and 2 are from tests with a product according to the invention with powder coating as described in Example 2, and curves 3 and 4 are from ordinary coating without powder as in WO Publication No. 81/01100.

In the test method used a dragee was placed over a slot whereupon it was cut through by means of a Verner-Bratxler knife at constant rate (50 mm/minute), said knife being a sheet of anodized aluminum with an angular recess where all the edges are deburred. The force necessary to obtain the constant rate of speed was constantly registered.

As mentioned, four tests were carried out, curves 1 and 2 illustrating tests with products according to the invention and curves 3 and 4 illustrate tests with products as described in WO Publication No. 81/01100. It can generally be stated that the steeper the curve until fracture, the stronger the perception of a "glass-like" hard surface, and that a more rounded or indented curve illustrates the perception of a very "grainy" surface. The clearer fracture of the curve, the clearer the perception of a hard glass-like dragee shell, that is a feeling in the mouth of a very crunchy piece of chewing gum.

As appears from the Figure, curves 1 and 2 are almost identical with homogenous coating and clear crunch and probably a good adhesion to the core. Curve 3 shows a fairly crisp, vaguely hard outer shell with a fair adhesion to the core and curve 4 shows a crisp, not particularly hard dragee shell and presumably poor adhesion to the core.

## Claims

1. A method for applying a hard sorbitol coating to cores of edible or chewable material where the cores in a moving bed in a coating apparatus are subjected to a number of coating cycles, each of the cycles comprising applying, distributing and smoothing out sorbitol-containing material and drying by means of drying air, whereupon the coated cores are optionally polished, **characterised** in that in succession the coating cycles comprise:

   1) a sealing wherein the cores are subjected to 1 to 6 cycles, each comprising the following steps (a), (c) and (d) :
   step (a), applying a sorbitol-containing suspension having a temperature of 15-25°C and containing at least 83% by weight of D-sorbitol based on dry matter,
   step (c) distributing and smoothing out the applied material on the surface of the cores,
   step (d), drying the material deposited on the cores using drying air having a temperature of 20-40°C and a relative humidity of 8-30%,
   2) a forced hard coating, wherein the cores are subjected to one or more cycles, each comprising the steps (a), (b), (c) and (d), each step (a), (c) and (d) being as mentioned above and step (b) comprising the application of a dry sorbitol powder with a particle size of between 40 micrometer and 125 micrometer and a D-sorbitol content of at least 94% by weight based on dry matter, and
   3) a hard coating, wherein the cores are subjected to one or more cycles, each comprising the above steps (a), (c) and (d).

2. The method as claimed in claim 1, **characterised** in that the coated cores are polished immediately after the coating cycles without intermediate conditioning of the coated cores.

3. The method as claimed in claim 2, **characterised** in that the polishing is carried out in the same coating apparatus as used for the coating.

4. The method as claimed in claim 1, **characterised** in that said sealing is carried out by completing three cycles each comprising the steps (a), (c) and (d).

5. The method as claimed in claim 1, **characterised** in that step (d) is carried out using drying air having a temperature of 26-30°C and a relative humidity of 9-15% in a quantity of 25-95 m$^3$/hour per m$^2$ of the surface area of the cores.

6. The method as claimed in claim 1, **characterised** in that the sorbitol-containing suspension used in step (a) has a temperature of 18-22°C.

7. The method as claimed in claim 1, **characterised** in that the method is carried out in a slowly rotating cylinder shaped coating apparatus with mixing blades, said coating apparatus being provided with perforations in the cylinder shaped surface of the coating apparatus for drying air feed.

8. The method as claimed in claim 1, **characterised** in that during said sealing (1) the drying step (d) is carried out by blowing drying air in at the bottom of the coating apparatus and up through the bed of cores, that during said forced hard coating (2), step (d) is carried out by blowing the drying air in from the top of the coating apparatus and down through the bed of cores, and that during said hard coating (3) step (d) is carried out by blowing the drying air in at the bottom of the coating apparatus and up through the bed of cores.

9. A dragee in the form of an edible or chewable core with a hard sorbitol coating, **characterised** in that it is prepared according to the method as claimed in any of the claims 1 to 8 above.

10. A dragee as claimed in claim 9, **characterised** in that the core is of a material selected among the group consisting of chewing gum, pastilles, snack, caramel, chocolate, dried fruits, dried vegetables, fruit bars, nuts, seeds and a medical products.

**Patentansprüche**

1. Verfahren zum Aufbringen einer harten Sorbitol-Beschichtung auf die inneren Teile von eßbarem oder kaubarem Material, in welchem die inneren Teile in einem bewegten Bett in einer Beschichtungs-Vorrichtung einer Reihe von Beschichtungs-Zyklen unterworfen werden, wobei jeder Zyklus umfaßt, Sorbitol enthaltendes Material aufzubringen, zu verteilen, auszuglätten und mittels Trocknungsluft zu trocknen, worauf die beschichteten inneren Teile wahlweise poliert werden, dadurch gekennzeichnet, daß die Beschichtungszyklen in der Reihenfolge umfassen:

1) Versiegeln, worin die inneren Teile 1 bis 6 Zyklen unterworfen werden, von denen jeder die folgenden Schritte (a), (c) und (d) umfaßt:

   Schritt (a), Auftragen einer Sorbitol enthaltenden Suspension, die eine Temperatur von 15-25°C besitzt und wenigstens 83 Gew.-% D-Sorbitol, basierend auf Trockensubstanz, enthält,

   Schritt (c), Verteilen und Ausglätten des aufgetragenen Materials auf der Oberfläche der inneren Teile,

   Schritt (d), Trocknen des auf den inneren Teilen abgelagerten Materials mittels Trocknungsluft, die eine Temperatur von 20-40°C und eine relative Feuchtigkeit von 8-30% besitzt,

2) eine verstärkte Hartbeschichtung, in der die inneren Teile einem oder mehreren Zyklen unterworfen werden, von denen jeder die Schritte (a), (b), (c) und (d) umfaßt, wobei jeder der Schritte (a), (c) und (d) wie vorstehend erwähnt ist, und Schritt (b) umfaßt, das Aufbringen eines trockenen Sorbitol-Pulvers mit einer Teilchengröße zwischen 40 μm und 125 μm und einen Gehalt an D-Sorbitol von wenigstens 94 Gew.-%, bezogen auf die Trockensubstanz, und

3) eine Hartbeschichtung, in der die inneren Teile einem oder mehreren Zyklen unterworfen werden, von denen jeder die vorstehenden Schritte (a), (c) und (d) umfaßt.

2. Verfahren nach Anspruch 1, dadurch gekennzeichnet, daß die beschichteten inneren Teile unmittelbar nach den Beschichtungszyklen ohne zwischenzeitliches Konditionieren der beschichteten inneren Teile poliert werden.

3. Verfahren nach Anspruch 2, dadurch gekennzeichnet, daß das Polieren in der gleichen Beschichtungs-Vorrichtung durchgeführt wird, die für das Beschichten verwendet wurde.

4. Verfahren nach Anspruch 1, dadurch gekennzeichnet, daß das Versiegeln durch Ausführen von drei Zyklen durchgeführt wird, von denen jeder die Schritte (a), (c) und (d) umfaßt.

5. Verfahren nach Anspruch 1, dadurch gekennzeichnet, daß Schritt (d) unter Verwendung von Trocknungsluft mit einer Temperatur von 26-30°C und einer relativen Feuchtigkeit von 9-15% in einer Menge von 25-95 m³/Stunde pro m² des Oberflächenbereichs der inneren Teile durchgeführt wird.

6. Verfahren nach Anspruch 1, dadurch gekennzeichnet, daß die in Schritt (a) verwendete, Sorbitol enthaltende Suspension eine Temperatur von 18-22°C besitzt.

7. Verfahren nach Anspruch 1, dadurch gekennzeichnet, daß das Verfahren in einer langsam rotierenden zylinderförmigen Beschichtungs-Vorrichtung mit Rührschaufeln durchgeführt wird, wobei die Beschichtungs-Vorrichtung mit Lochungen in der zylinderförmigen Oberfläche der Beschichtungs-Vorrichtung versehen ist, um Trocknungsluft zu liefern.

8. Verfahren nach Anspruch 1, dadurch gekennzeichnet, daß der Trocknungschritt (d) während des Versiegelns (1) (d) durchgeführt wird, durch Einblasen der Trocknungsluft am Boden in die Beschichtungs-Vorrichtung und durch das Bett der inneren Teile nach oben,

   daß Schritt (d), während der verstärkten Hartbeschichtung (2) durchgeführt wird durch Einblasen der Trocknungsluft von der Spitze der Beschichtungs-Vorrichtung und durch das Bett der inneren Teile nach unten, und

   daß Schritt (d), während der Hartbeschichtung (3) durchgeführt wird durch Einblasen der Trocknungsluft am Boden der Beschichtungs-Vorrichtung und durch das Bett der inneren Teile nach oben.

**9.** Dragee in Form eines eßbaren oder kaubaren inneren Teils mit einer harten Sorbitol-Beschichtung, dadurch gekennzeichnet, daß es nach einem Verfahren der Ansprüch 1 bis 8 hergestellt wurde.

**10.** Dragee nach Anspruch 9, dadurch gekennzeichnet, daß das innere Material ein Material ist ausgewählt aus der Gruppe bestehend aus Kaugummi, Pastillen, Imbiss, Karamel, Schokolade, getrockneten Früchten, getrocknetem Gemüse, Früchteriegeln, Nüssen, Samen und medizinischen Produkten.

**Revendications**

**1.** Procédé pour appliquer un revêtement de sorbitol dur sur des noyaux de substance comestible ou masticable, dans lequel les noyaux situés dans un lit mobile à l'intérieur d'un appareil de revêtement sont soumis à un certain nombre de cycles de revêtement, chacun des cycles comprenant l'application, la distribution et le lissage d'une substance contenant du sorbitol et le séchage par de l'air de séchage, après quoi les noyaux revêtus sont éventuellement polis, caractérisé en ce que les cycles de revêtement comprennent successivement:

1) un enrobage dans lequel les noyaux sont soumis à 1 à 6 cycles comprenant chacun les étapes (a), (c) et (d) suivantes:

étape (a) : application d'une suspension contenant du sorbitol ayant une température de 15 à 25°C et contenant au moins 83 % en poids de D-sorbitol par rapport à la matière sèche,

étape (c) : distribution et lissage de la substance appliquée sur la surface des noyaux,

étape (d) : séchage de la substance déposée sur les noyaux par de l'air de séchage ayant une température de 20 à 40°C et une humidité relative de 8 à 30 %,

2) un revêtement dur forcé dans lequel les noyaux sont soumis à un ou plusieurs cycles comprenant chacun les étapes (a), (b), (c) et (d), chaque étape (a), (c) et (d) étant telle que mentionnée ci-dessus et l'étape (b) comprenant l'application d'une poudre de sorbitol sèche ayant une taille de particules comprise entre 40 µm et 125 µm et une teneur en D-sorbitol d'au moins 94 % en poids par rapport à la matière sèche, et

3) un revêtement dur dans lequel les noyaux sont soumis à un ou plusieurs cycles comprenant chacun les étapes (a), (c) et (d) ci-dessus.

**2.** Procédé selon la revendication 1, caractérisé en ce que les noyaux revêtus sont polis immédiatement après les cycles de revêtement sans conditionnement intermédiaire des noyaux revêtus.

**3.** Procédé selon la revendication 2, caractérisé en ce que le polissage est réalisé dans le même appareil de revêtement que celui qui est utilisé pour le revêtement.

**4.** Procédé selon la revendication 1, caractérisé en ce que ledit enrobage est réalisé par l'accomplissement de trois cycles comprenant chacun les étapes (a), (c) et (d).

**5.** Procédé selon la revendication 1, caractérisé en ce que l'étape (d) est réalisée avec de l'air de séchage ayant une température de 26 à 30°C et une humidité relative de 9 à 15 % en une quantité de 25 à 95 m³/h par m² de surface des noyaux.

**6.** Procédé selon la revendication 1, caractérisé en ce que la suspension contenant du sorbitol utilisée dans l'étape (a) a une température de 18 à 22°C.

**7.** Procédé selon la revendication 1, caractérisé en ce que le procédé est mis en oeuvre dans un appareil de revêtement en forme de cylindre à rotation lente muni de pales mélangeuses, ledit appareil de revêtement étant muni de perforations dans la surface en forme de cylindre de l'appareil de revêtement pour l'introduction de l'air de séchage.

**8.** Procédé selon la revendication 1, caractérisé en ce que, pendant ledit enrobage (1), l'étape de séchage (d) est accomplie par insufflation d'air de séchage au fond de l'appareil de revêtement et dans le sens ascendant à travers le lit de noyaux, en ce que, pendant ledit revêtement dur forcé (2), l'étape (d) est accomplie par insufflation de l'air de séchage par le sommet de l'appareil de revêtement et dans le sens descendant à travers le lit de noyaux, et en ce que, pendant ledit revêtement dur (3), l'étape (d) est accomplie par insufflation de l'air de séchage au fond de l'appareil de revêtement et dans le sens ascendant à travers le lit de noyaux.

9. Dragée sous forme d'un noyau comestible ou masticable muni d'un revêtement de sorbitol dur, caractérisée en ce qu'elle est préparée par le procédé selon l'une quelconque des revendications 1 à 8 ci-dessus.

10. Dragée selon la revendication 9, caractérisée en ce que le noyau est constitué par une substance choisie dans le groupe formé par la gomme à mâcher, les pastilles, les aliments pour restauration rapide, le caramel, le chocolat, les fruits séchés, les légumes secs, les barres aux fruits, les noix, les graines et les produits médicaux.

# Fig.1